Europäisches Patentamt

**European Patent Office**

Office européen des brevets

(11) Veröffentlichungsnummer: **0 018 668**
**A2**

# (12) EUROPÄISCHE PATENTANMELDUNG

(21) Anmeldenummer: **80102529.7**

(22) Anmeldetag: **08.05.80**

(51) Int. Cl.³: **A 61 K 7/155**

(30) Priorität: **08.05.79 DE 2918422**

(43) Veröffentlichungstag der Anmeldung:
**12.11.80 Patentblatt 80/23**

(84) Benannte Vertragsstaaten:
**AT BE CH FR GB IT LI LU NL SE**

(71) Anmelder: **Kasidecioglu, Ertas**
**Handwerkerwall 29**
**D-3170 Gifhorn(DE)**

(72) Erfinder: **Kasidecioglu, Ertas**
**Handwerkerwall 29**
**D-3170 Gifhorn(DE)**

(74) Vertreter: **Gralfs, Harro, Dipl.-Ing.**
**Am Bürgerpark 8**
**D-3300 Braunschweig(DE)**

(54) **Mittel zur mechanischen Entfernung von Körperhaaren.**

(57) Mittel zur mechanischen Entfernung von Körperhaaren in Form einer Mischung aus Zucker, Wasser, einer schwachen Säure und Harz, mit der Zusammensetzung:
    100 Gewichtsteile Zucker
    18 bis 20 Gewichtsteile Wasser
    2 bis 6 Gewichtsteile Gummi Arabicum
    0,2 bis 0,4 Gewichtsteile Citronensäure, Weinsäure oder eine andere physiologisch unbedenkliche Säure.

EP 0 018 668 A2

Croydon Printing Company Ltd.

Ertas Kasidecioglu
Handwerkerwall 29
3170 Gifhorn
Bundesrepublik Deutschland

Mittel zur mechanischen Entfernung von Körperhaaren

Die Erfindung betrifft ein Mittel zur mechanischen Entfernung von Körperhaaren in Form einer Mischung aus Zucker, Wasser, einer schwachen Säure und Harz.

Bei einem bekannten Haarentfernungsmittel der genannten Art (DE-PS 1 036 472) enthält die Mischung Wasser mit einem Gewichtsanteil, der doppelt so hoch ist wie der Gewichtsanteil des Zuckers, und sie hat einen relativ hohen Gewichtsanteil von Wachs oder Fett. Das Mittel ist pastös und muß nach dem Aufbringen auf die Haut zunächst trocknen. Die Behandlung ist damit auch bei relativ schneller Trocknung zeitaufwendig und der Erfolg ist abhängig davon, daß ein ausreichender Trocknungsgrad erreicht wird.

Wachse und Fette, die in den meisten bekannten Haarentfernungsmitteln enthalten sind, sind physiologisch nicht unbedenklich, da sie auch bei äußerlicher Anwendung Allergien hervorrufen können. Das gleiche gilt für viele Harze, insbesondere für das vielfach in bekannten Haarentfernungsmitteln enthaltene Kolophonium.

Aufgabe der Erfindung ist es, ein Haarentfernungsmittel zu schaffen, das bei der Anwendung keiner Trocknung bedarf und mit dem damit Behandlungsfehler weitgehend ausgeschlossen sind und das weiterhin physiologisch unbedenklich ist, insbesondere keine Bestandteile enthält, die zu Allergien führen könnten.

Diese Aufgabe wird gemäß der Erfindung gelöst durch ein Haarentfernungsmittel, das gekennzeichnet ist durch folgende Zusammensetzung:

100 Gewichtsteile Zucker
18 bis 20 Gewichtsteile Wasser
2 bis 6 Gewichtsteile Gummi Arabicum
0,2 bis 0,4 Gewichtsteile Citronensäure, Weinsäure oder eine andere physiologisch unbedenkliche Säure.

Wasser wird nur in einer solchen Menge verwendet, die ausreicht, den Zucker zu lösen und mit Hilfe der Citronensäure so anzusäuern, daß keine Rekristallisation eintritt. Das fertige Produkt ist weitgehend wasserfrei. Es trocknet damit in Tuben oder Gefäßen auch nicht ein.

Der Anteil des Gummi Arabicum ist nach oben nicht kritisch begrenzt. Es können also auch höhere Gewichtsanteile verwendet werden bei entsprechender Erhöhung des Wasseranteils um die zu seinem Lösen benötigte Menge. Ein Mehr an Gummi Arabicum führt jedoch nicht zu einer wesentlichen Verbesserung des Mittels.

Bei der Herstellung wird zunächst die Säure in das Wasser eingegeben und das Wasser dann auf etwa 60 bis 80° erwärmt. Anschließend wird der Zucker zugegeben unter gleichzeitiger Erhöhung der Temperatur auf etwa 105 bis 120°. Das Gummi Arabicum wird in einem Teil des Wassers gelöst und dann der sauren Zuckerlösung hinzugefügt. Unter fortgesetztem Rühren läßt man die Mischung dann auf etwa 70° C abkühlen. Sie kann dann in Tuben oder sonstige Behälter abgefüllt werden.

Nach dem Erkalten hat das Gemisch eine zähe hochviskose Konsistenz. Bei der Anwendung wird das Mittel auf die Haut aufgespachtelt und zwar in Richtung des natürlichen Haarwuchses. Auf das aufgespachtelte Haarentfernungsmittel wird dann ein Stoffstreifen aufgedrückt, der dann entgegen der Richtung, in der das Mittel aufgespachtelt wurde, abgezogen wird. Das Abziehen kann sofort erfolgen. Dabei löst sich das Mittel rückstandslos von der Haut und nimmt praktisch alle Haare mit. Die Enthaarung erfolgt damit ohne Wartezeiten. Da das Mittel nur wasserlösliche Bestandteile enthält, genügt eine Nachbehandlung mit Wasser.

Bei bekannten Haarentfernungsmitteln, die Wachse und Fette enthalten, ist vielfach eine Reinigung mit Lösungsmitteln erforderlich, die zu Schädigungen der Haut führen können, die durch das Herausziehen der Haare aus ihren Kapillaren ohnehin irritiert ist.

- 1 -

<u>A n s p r u c h</u>

Mittel zur mechanischen Entfernung von Körperhaaren in Form einer Mischung aus Zucker, Wasser, einer schwachen Säure und Harz, <u>gekennzeichnet durch</u> folgende Zusammensetzung:

100 Gewichtsteile Zucker

18 bis 20 Gewichtsteile Wasser

2 bis 6 Gewichtsteile Gummi Arabicum

0,2 bis 0,4 Gewichtsteile Citronensäure, Weinsäure oder eine andere physiologisch unbedenkliche Säure.